# FASCICULE DE BREVET EUROPEEN

(11) **EP 2 190 884 B1**
(45) Date de publication et mention de la délivrance du brevet: **20.07.2011**
(21) Numéro de dépôt: 08804024.1
(22) Date de dépôt: 11.09.2008
(51) Int. Cl.: C08B 37/00, C08G 65/333, C08G 81/00, A61K 8/73, A61K 8/91, A61K 31/722, A61P 17/02, A61L 27/20, A61L 27/52

(54) **PROCÉDÉ DE PRÉPARATION DE DÉRIVÉS (POLY(OXYDE D'ÉTHYLÈNE) POLY(OXYDE DE PROPYLÈNE)) THERMOSENSIBLES UTILES POUR FONCTIONNALISER LE CHITOSANE.**
VERFAHREN ZUR HERSTELLUNG VON WÄRMEEMPFINDLICHEN (POLY(ETHYLENOXID)-POLY(PROPYLENOXID))-DERIVATEN, DIE ZUR FUNKTIONALISIERUNG VON CHITOSAN VERWENDET WERDEN KÖNNEN
PROCESS FOR PREPARING THERMOSENSITIVE (POLY(ETHYLENE OXIDE) POLY(PROPYLENE OXIDE)) DERIVATIVES THAT CAN BE USED TO FUNCTIONALIZE CHITOSAN.

(30) Priorité: 11.09.2007 FR 0757506
(43) Date de publication de la demande: 02.06.2010
(73) Titulaire: Centre National de la Recherche Scientifique (CNRS), 75016 Paris (FR); Universite Joseph Fourier - Grenoble 1, 38400 St. Martin d'Heres (FR)
(72) Inventeur: AUZELY-VELTY, Rachel, F-38450 Le Gua (FR); CREUZET, Caroline, F-38610 Gieres (FR)
(74) Mandataire: Warcoin, Jacques
(86) Numéro de dépôt international: PCT/EP2008/062059
(87) Numéro de publication internationale: WO 2009/034130

(56) Documents cités:
- WO-A-03/049699
- US-A- 5 990 237
- US-A1- 2003 129 730
- US-A1- 2004 166 158
- CREUZET C., AUZÉLY-VELTY R., RINAUDO M.: "Synthèse et étude d'hydrogels thermosensibles obtenus par modification chimique contrôlée du chitosane" L'ACTUALITÉ CHIMIQUE, vol. 294, février 2006 (2006-02), pages 34-38, XP001537956 cité dans la demande
- HOFFMAN A S ET AL: "GRAFT COPOLYMERS OF PEO-PPO-PEO TRIBLOCK POLYETHERS ON BIOADHESIVE POLYMER BACKBONES: SYNTHESIS AND PROPERTIES" POLYMER PREPRINTS, AMERICAN CHEMICAL SOCIETY, US, vol. 38, no. 1, 1997, pages 524-525, XP008014013 ISSN: 0032-3934
- BHATTARAI N ET AL: "PEG-grafted chitosan as an injectable thermosensitive hydrogel for sustained protein release" JOURNAL OF CONTROLLED RELEASE, ELSEVIER, AMSTERDAM, NL, vol. 103, no. 3, 18 avril 2005 (2005-04-18), pages 609-624, XP004831754 ISSN: 0168-3659 cité dans la demande

## Description

L'invention concerne de nouveaux polymères thermosensibles, qui peuvent notamment être utilisés dans la synthèse de dérivés du chitosane.

Les hydrogels réversibles formés in situ suite à une augmentation de température suscitent actuellement un intérêt croissant en raison de leurs nombreuses applications potentielles dans les domaines cosmétique, pharmaceutique et biomédical. Dans ces systèmes, la formation des jonctions physiques nécessaires à la gélification est généralement due à l'association de polymères possédant une température critique inférieure de démixtion. Ces polymères dits à LCST (Lower Critical Solution Temperature) conduisent à une séparation de phase lorsque la température devient supérieure à la LCST. En effet, ces systèmes voient leur hydrophobie augmenter avec la température. Comme exemples, on peut citer le poly(N-isopropylacrylamide) (PNIPAM) et les polyéthers triblocs de poly(oxyde d'éthylène) (POE) et de poly(oxyde de propylène) (POP) commercialisés sous le nom de Pluronics® (BASF) ou Poloxamers® (ICI) (Gil et al. 2004). Les triblocs POE-POP-POE ont la propriété de former des gels à haute température ; or pour cela, ils nécessitent d'être à des concentrations en solution très élevées (de 200 à 300 g/L), ce qui ne constitue pas des conditions optimales pour la biocompatibilité. L'une des stratégies possibles pour pallier ce problème repose sur le greffage à un faible taux de ces polymères sur un biopolymère, tel que le chitosane. Cette approche présente plusieurs avantages ; un comportement thermogélifiant peut être observé en milieu aqueux avec de faibles concentrations en polymère (10 g/L), combinant par ailleurs les propriétés de biocompatibilité, biodégradabilité et biologiques du chitosane. Ce polysaccharide d'origine naturelle augmente la vitesse de cicatrisation de plaies ouvertes, en stimulant la réponse immunitaire et la reconstruction des tissus, en prévenant les infections microbiennes et en absorbant l'exudat. Il s'avère également être un bon substrat pour la culture cellulaire et stimule, de plus, la croissance cellulaire. Ces propriétés en font donc un bon candidat pour la cicatrisation des plaies, la médecine régénératrice (reconstruction osseuse) et l'élaboration de systèmes d'administration de médicaments (implants, solutions, hydrogels, patchs).

Dans ce contexte, les inventeurs ont développé des systèmes thermogélifiants par greffage de polymères à LCST sur le chitosane. Ces systèmes consistent en des solutions aqueuses dont le pH varie avantageusement de 4 à des valeurs de pH physiologique, conduisant à des gels transparents par chauffage.
Peu de travaux ont été consacrés au développement d'hydrogels thermoréversibles à base de polymères naturels. Chenite et al. (Biomaterials (2000), 21 2155-2161) ont montré la possibilité d'obtenir des systèmes thermogélifiants à partir de solutions aqueuses à pH physiologique de chitosane et de sels de glycerol-2-phosphate. Néanmoins, l'obtention de ces gels nécessite l'utilisation d'une quantité importante de sels qui n'autorise pas leur utilisation dans certaines applications biomédicales. Des dérivés du chitosane porteurs de chaînes de PNIPAM ont également été développés dans le but de les utiliser en ingénierie tissulaire. (Cho et al. Biomaterials (2004), 25,5743-5751). Cependant, le PNIPAM ne présente pas les caractéristiques de biocompatibilité requises pour de réelles applications in vivo. Chung et al. (Curr. Applied Phys. (2005), 5, 485-488) ont décrit la synthèse de dérivés du chitosane thermoépaississants porteurs de chaînes de type Pluronics®, biocompatibles. Mais la nature bifonctionnelle de telles chaînes rend le contrôle de la synthèse relativement difficile.
Le poly(oxyde d'éthylène) poly(oxyde de propylène), POEPOP, appelé commercialement Jeffamine® (Huntsman), est un copolymère à arrangement aléatoire d'unités oxyde d'éthylène et oxyde de propylène de la famille des Pluronics®. Ce copolymère biocompatible avait été utilisé par L'Alloret (FR 2 811 995) pour préparer des copolymères greffés thermogélifiants dérivés de polymères synthétiques ou de dérivés de polysaccharides autres que le chitosane (cellulose et dérivés, galactomannanes, alginates). Ce polymère a également été utilisé pour fonctionnaliser le chitosane. Le chitosane possède une fonction amine en position C-2, permettant d'effectuer des réactions régiosélectives. La synthèse du chitosane-POEPOP repose sur la fonctionnalisation du POEPOP par un sucre réducteur dans une première étape (réaction de couplage du type peptidique entre le POEPOP et l'acide galacturonique protégé suivie de la déprotection des hydroxyles du sucre, conduisant au dérivé POEPOP-GA), suivie dans une seconde étape du couplage du POEPOP modifié avec le chitosane par une réaction d'amination réductrice. Le carbone C-1 du sucre, de par son caractère hémiacétalique, peut réagir avec la fonction amine du chitosane (Creuzet et al., l'actualité chimique (2006), 294, 34-38). Toutefois, ce procédé ne permet pas de produire de quantités importantes (industrielles) de polysaccharides modifiés ; le dérivé POEPOP-GA étant de synthèse difficile à mettre en oeuvre et coûteuse.
Les inventeurs ont ainsi développé un nouveau procédé de synthèse via un nouveau dérivé, le POEPOP-acétal, pour conduire à des dérivés CHI-POEPOP de degré de substitution (DS) variable, avantageusement solubles en milieu aqueux à pH 4 et thermogélifiants. La température de transition sol-gel dépend du degré de substitution, ce qui permet d'ajuster les propriétés en fonction des applications souhaitées. De façon à élargir les applications, des dérivés CHI-POEPOP-POE, avantageusement solubles en milieu aqueux à des valeurs de pH physiologique, ont été par ailleurs développés à partir de l'intermédiaire POEPOP-acétal ou du dérivé CHI-POEPOP.

L'invention a donc pour premier objet un procédé de préparation de dérivé POEPOP-acétal thermosensible comprenant les étapes successives suivantes :
a. Réaction d'un ou plusieurs polyétheramines statistiques (POEPOP) de formule générique (I) 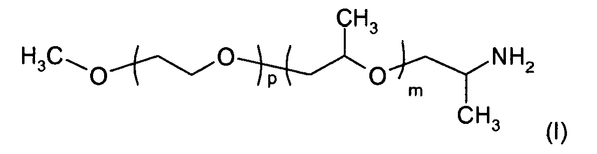 dans lequel p représente un nombre entier variant de 1 à 40, avantageusement de 3 à 29, et m représente un nombre entier variant de 1 à 40, avantageusement de 1 à 31, avec l'anhydride succinique pour conduire à l'acide correspondant;
b. Couplage de l'acide obtenu suite à l'étape a) avec la 2,2-diméthoxyéthylamine pour conduire au dérivé POEPOP-acétal recherché. Dans le cadre de la présente invention, les abréviations POEPOP font référence à un ou plusieurs copolymères statistiques d'oxyde éthylène et d'oxyde de propylène. Dans le cadre du procédé selon l'invention, il est possible d'utiliser soit un seul copolymère statistique d'oxyde d'éthylène et d'oxyde de propylène, soit un mélange de différents copolymères statistiques d'oxyde d'éthylène et d'oxyde de propylène, lesdits copolymères pouvant notamment différer de part le ratio oxyde de propylène /oxyde d'éthylène et le poids moléculaire.

Les polyétheramines statistiques utilisés dans le cadre de la présente invention sont avantageusement des copolymères commercialisés par Huntsman sous la dénomination commerciale Jeffamine®, en particulier les copolymères statistiques:
- de masse moléculaire approximative de 600 g/mol, le ratio oxyde de propylène /oxyde d'éthylène étant de 9/1;
- de masse moléculaire approximative de 1000 g/mol, le ratio oxyde de propylène /oxyde d'éthylène étant de 3/19 ;
- de masse moléculaire approximative de 2000 g/mol, le ratio oxyde de propylène /oxyde d'éthylène étant de 29/6 ; et
- de masse moléculaire approximative de 2000 g/mol, le ratio oxyde de propylène /oxyde d'éthylène étant de 10/31.

Outre les produits disponibles dans le commerce, les polyétheramines statistiques peuvent être facilement préparés par des procédés conventionnels biens connus de l'homme de l'art (Y. Deng, J. Ding, G. Yu, R. H. Mobbs, F. Heatley, C. Price, C. Booth, Plmer (1992), 33, 1959-1962; G. -E. Yu, F. Heatley, C. Booth, T G. Blease, Eur. Polym. J. (1995), 31, 589-593).

On sait que la répartition statistique des motifs oxyde d'éthylène et oxyde de propylène se traduit par l'existence d'une température inférieure critique de démixtion, au delà de laquelle une séparation de phases macroscopique est observée.

Dans le cadre de l'invention, l'expression « dérivé thermosensible » désigne un polymère à LCST, c'est-à-dire un polymère dont la solubilité dans l'eau est modifiée au-delà d'une certaine température. Il s'agit de polymères présentant une température de demixtion par chauffage (ou point de trouble) définissant leur zone de solubilité dans l'eau. La température de demixtion minimale obtenue en fonction de la concentration en polymère est appelée LCST. Pour chaque concentration en polymère, cette température de demixtion par chauffage est observée; elle est supérieure à la LCST qui est le point minimum de la courbe. En dessous de cette température, le polymère est soluble dans l'eau; au dessus de cette température, le polymère perd sa solubilité dans l'eau.

Par soluble dans l'eau, on entend que les unités présentent une solubilité à 20 °C, d'au moins 1 g/l, de préférence au moins 2 g/l.

La mesure de la LCST peut se faire visuellement : on détermine la température à laquelle apparaît le point de trouble de la solution aqueuse ; le point de trouble se traduit par l'opacification de la solution, ou la perte de transparence.

D'une manière générale, une composition transparente aura une valeur de transmittance maximum de la lumière, quelque soit la longueur d'onde comprise entre 400 et 800 nm, à travers un échantillon de 1 cm d'épaisseur, d'au moins 80%, de préférence d'au moins 90% (voir EP291334). La transmittance peut être mesurée en plaçant un échantillon de 1 cm d'épaisseur dans le rayon lumineux d'un spectrophotomètre travaillant dans les longueurs d'onde du spectre lumineux.
Le procédé est avantageusement réalisé au moins partiellement dans des conditions anhydres, afin d'assurer de bons rendements. Comme exemple de solvant particulièrement approprié pour la conduite de l'étape a), on peut citer le diméthylformamide (DMF), en particulier le DMF anhydre.
Dans un mode de réalisation préféré de l'invention, à une solution de POEPOP, dans le DMF, avantageusement le DMF anhydre, est ajouté l'anhydride succinique en solution dans le DMF, avantageusement le DMF anhydre. La solution de POEPOP /DMF (anhydre) est avantageusement maintenue sous atmosphère inerte, tel que sous azote. La quantité molaire nécessaire d'anhydride succinique est au minimum la quantité molaire de POEPOP, avantageusement l'anhydride succinique est introduit en léger excès (avantageusement 1,05 équivalents molaires).
Le solvant utilisé à l'étape b) est avantageusement également du DMF, en particulier le DMF anhydre.
Dans l'étape b), la réaction de couplage est avantageusement effectuée en présence de 1-éthyl-3-[3-(diméthylamino)propyl]-carbodiimide et de diisopropyléthylamine (ou de triéthylamine). Ainsi, l'étape b) comprend avantageusement l'ajout successif
- de 1-éthyl-3-[3-(diméthylamino)propyl]-carbodiimide et de la diisopropyléthylamine (ou de triéthylamine), solubilisé dans le DMF anhydre, puis
- de 2,2-diméthoxyéthylamine.
Il est également possible d'ajouter préalablement du N-hydroxybenzotriazole. L'étape b) comprend alors avantageusement l'ajout successif
- de N-hydroxybenzotriazole, puis
- de 1-éthyl-3-[3-(diméthylamino)propyl]-carbodiimide et de la diisopropyléthylamine (ou de triéthylamine), solubilisé dans le DMF anhydre, puis
- de 2,2-diméthoxyéthylamine.

Le procédé selon l'invention permet la réalisation des étapes a) et b) « en un seul pot », c'est-à-dire dans un seul et même réacteur, sans étape intermédiaire d'isolement du composé obtenu suite à l'étape a). Les étapes a) et b) sont donc avantageusement réalisées « en un seul pot ».
Pour s'assurer que, préalablement à la réaction de couplage, la réaction du POEPOP avec l'anhydride succinique est terminée, le milieu réactionnel est laissé sous agitation le temps nécessaire. Le milieu réactionnel peut être laissé ainsi sous agitation à température ambiante et à pression atmosphérique. A l'échelle du laboratoire (<500mL), le milieu réactionnel est avantageusement laissé sous agitation à température ambiante et à pression atmosphérique pendant 5 heures.
Suite à l'étape b), le milieu réactionnel est avantageusement laissé sous agitation à température ambiante et à pression atmosphérique le temps nécessaire pour s'assurer que la réaction de couplage est terminée, ce dont on s'assure, à l'échelle du laboratoire (<500 mL) en laissant la solution sous agitation toute une nuit.
Pour récupérer le POEPOP-acétal, on peut avantageusement opérer de la manière suivante. Le solvant (DMF) est évaporé et le produit est repris dans un solvant approprié tel que le dichlorométhane. La phase organique est lavée successivement avec de l'eau acidulée (pH 4), puis de l'eau. La phase organique est séchée, par exemple séchage sur sulfate de sodium, puis évaporée pour donner le dérivé acétal. Le procédé selon l'invention permet d'obtenir le dérivé POEPOP-acétal avec des rendements supérieurs à 80%.
Dans une variante de l'invention, on fait réagir, à l'étape a), un seul polyétheramine statistique. Dans une autre variante de l'invention, on fait réagir, à l'étape a), au moins deux polyétheramines statistiques différents.

L'invention a pour deuxième objet un dérivé POEPOP-acétal obtenu par le procédé, les abréviations POEPOP désignant des copolymères statistiques d'oxyde d'éthylène et d'oxyde de propylène, identiques ou différents, tels que définis précédemment.

L'invention a pour troisième objet un procédé de synthèse de chitosane greffé comprenant (i) une étape d'hydrolyse du dérivé POEPOP-acétal selon l'invention en dérivé POEPOP-CHO (c'est-à-dire en l'aldéhyde correspondant) suivie (ii) d'une étape de greffage dudit dérivé POEPOP-CHO sur le chitosane ou un des ses dérivés par une réaction du type amination réductrice.
La première étape (i) consiste à déprotéger la fonction aldéhyde du POEPOP-acétal. Pour ce faire, à l'échelle du laboratoire (<500 mL), celui-ci est avantageusement solubilisé dans un mélange acide trifluoroacétique / eau (TFA/H₂O), par exemple dans les proportions (4/1) (v/v). Le milieu réactionnel peut être laissé sous agitation pendant une nuit à température ambiante et à pression atmosphérique. Après évaporation du mélange TFA/H₂O, l'aldéhyde obtenu peut être solubilisé dans un mélange éthanol /eau (en particulier EtOH/ H₂O (1/1 (v/v)) et le pH de la solution est avantageusement ajusté à pH 5,1.
L'aldéhyde est alors ajouté au chitosane, qui peut préalablement être solubilisé dans un mélange CH₃COOH / EtOH (3 :2 v/v) par exemple. Le pH du mélange est alors ajusté à 5,1. Une solution aqueuse d'un agent réducteur, tel qu'une solution aqueuse de cyanoborohydrure de sodium ou de picoline borane (PicBH₃) ou de borohydrure de sodium, est avantageusement ajoutée. Le milieu réactionnel peut être laissé sous agitation le temps nécessaire, 24 heures à l'échelle du laboratoire (<500 mL), à température ambiante et à pression atmosphérique. Le produit attendu peut être récupéré par précipitation, en ajustant le pH du milieu réactionnel à 9. Ce dernier est alors lavé, filtré et séché.
Le chitosane ou un des ses dérivés répond avantageusement à la formule (II) suivante : 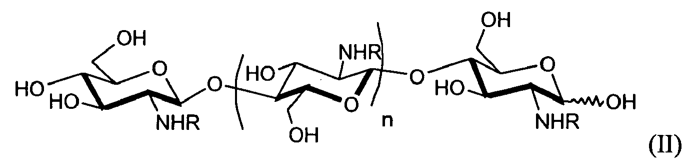 dans laquelle n varie de 60 à 6000 et R représente
- un atome d'hydrogène H,
- un radical acétyle COCH₃, le degré d'acétylation étant avantageusement compris entre 0 et 0,5, plus avantageusement entre 0 et 0,2.
Les inventeurs ont constaté que le degré de substitution du POEPOP sur le chitosane, DS₁, est ajustable en fonction des conditions de réaction utilisées et, en particulier, dépend de la concentration en aldéhyde (PEOPOP-CHO). Le tableau 1 rassemble les conditions de réactions utilisées pour la synthèse de différents échantillons de CHI-POEPOP de DS₁ variable ; le polyétheramine statistique ayant une masse moléculaire approximative de 2000 g/mol, le ratio oxyde de propylène /oxyde d'éthylène étant de 29/6.

**Tableau 1. Conditions de réaction utilisées pour la synthèse des dérivés CHI-POEPOP de DS₁ variable.**

| Réaction | NaCNBH₃^{a} | POEPOP-CHO^{a} | Rendement (%) | DS₁^{b} |
|---|---|---|---|---|
| 1 | 1,2 | 0,05 | 72 | 0,04 |
| 2 | 1,2 | 0,1 | 63 | 0,1 |
| 3 | 1,2 | 0,2 | 48 | 0,17 |

| | | | | |
|---|---|---|---|---|
| ^{a} Nombre d'équivalents molaires/ mole d'unité de répétition;^{b} obtenu par RMN 1H | | | | |

Les données du tableau 1 montrent que la réaction de greffage est parfaitement contrôlée. Elle permet d'aboutir à une famille de produits de DS₁ variable, ce qui permet d'ajuster la gamme de température pertinente pour l'application envisagée. Dans le cas du dérivé POEPOP testé (polymère relativement hydrophobe s'associant rapidement par chauffage), on constate que plus le degré de substitution DS₁ est élevé, plus la température de transition est faible. Ainsi, pour des valeurs de DS₁=0,04, la température de transition se situe aux alentours de 40°C alors que pour des valeurs de DS₁=0,17, la température de transition se situe aux alentours de 25°C.
Le degré de substitution du POEPOP sur le chitosane est avantageusement compris entre 0,02 et 0,3, plus avantageusement entre 0,05 et 0,2.
Les dérivés CHI-POEPOP obtenus par ce procédé présentent des propriétés thermogélifiantes intéressantes, à savoir lorsqu'ils sont mis en solution une transition sol-gel réversible et contrôlable. Ils sont en outre solubles dans l'eau à des pH proches de 4 (±1). Les dérivés de chitosane CHI-POEPOP peuvent être solubilisés dans des compositions aqueuses (pH de 4 ± 1), par exemple en une quantité comprise entre 2 et 30 g/L, qui peuvent par ailleurs comprendre un milieu cosmétiquement ou pharmaceutiquement acceptable. Les solutions aqueuses résultantes conduisent à des liquides hautement visqueux ou à des gels transparents par chauffage. Ces systèmes, biorésorbables, peuvent être utilisés dans des formulations cosmétiques, pharmaceutiques ou biomédicales, qui peuvent être transparentes, pour en modifier les propriétés rhéologiques en fonction de la température. La capacité de gélification des solutions aqueuses comprenant les dérivés CHI-POEPOP à des températures voisines de celles du corps humain peut permettre d'éviter des chirurgies, ces gels pouvant alors être injectés ou appliqués à des sites spécifiques. Ces thermogels présentent en outre l'avantage d'être biorésorbables. Ces solutions aqueuses peuvent donc être utilisées en tant qu'hydrogel injectable dans des applications thérapeutiques ou cosmétiques.

Afin d'élargir les applications, des dérivés CHI-POEPOP-POE thermogélifiants solubles en milieu aqueux à des valeurs de pH physiologique ont été développés à partir de l'intermédiaire POEPOP-acétal ou du dérivé CHI-POEPOP obtenu. Ces dérivés se caractérisent par la présence, en plus de chaînes POEPOP, de chaînes poly(oxyde d'éthylène) (POE) greffées directement sur le chitosane, qui confèrent au chitosane les propriétés de solubilité dans l'eau à pH neutre. Bhattarai et al. (Macromol. Biosci. (2005), 5, 107-11 et J. Controlled Release (2005), 103, 609-624) ont montré que l'introduction de chaîne POE sur le chitosane peut conduire à un comportement thermoassociatif si le DS n'est pas trop élevé (DS ~ 0,07-0,1). Pour des DS supérieurs à 0,1, les dérivés CHI-POE sont solubles dans l'eau mais ne montrent plus d'effet thermoépaississant. Les inventeurs ont constaté que la synthèse de dérivés CHI-POE de DS < 0,1 ne permet pas d'obtenir une parfaite solubilité des polymères dans l'eau. Par ailleurs, l'augmentation de la viscosité des solutions des dérivés CHI-POE de DS ~ 0,07-0,1 décrits par Bhattarai et al. reste modérée (augmentation d'un facteur 6 pour une solution aqueuse à 30 g/L). Au contraire, les dérivés CHI-POEPOP-POE selon l'invention permettent des gains en viscosité bien supérieurs.
Le procédé de synthèse de chitosane greffé comprend donc avantageusement une étape supplémentaire (iii) de fonctionnalisation du dérivé chitosane CHI-POEPOP obtenu suite à l'étape (ii) par un polymère, en particulier un poly(oxyde d'éthylène) (POE). Le POE peut avoir une masse moléculaire comprise entre 1000 et 5000 g/mol. Le degré de substitution du POE sur le chitosane, DS₂, doit être supérieur à 0,1 pour obtenir une parfaite solubilité des dérivés dans l'eau. Le degré de substitution du POE sur le chitosane, DS₂, est avantageusement compris entre 0,1 et 0,5, plus avantageusement entre 0,1 et 0,3.
Selon une variante de l'invention, on prépare d'abord un dérivé monoaldéhydique du POE. Pour cela, on peut ajouter lentement à une solution de monoéther méthylique de poly(oxyde d'éthylène) dans un solvant approprié, tel qu'une solution de CH₂Cl₂/diméthylsulfoxide (DMSO) anhydre (97/3 (v/v)), sous azote, une solution de Dess-Martin Periodinane (DMP) dans un solvant tel que le DMSO. Le mélange réactionnel peut être laissé sous agitation, par exemple pendant 2 heures à l'échelle du laboratoire (<500 mL), à température ambiante. Le milieu est ensuite concentré, le produit précipité, isolé et séché.
A une solution de chitosane-POEPOP, qui peut être préalablement solubilisé dans un mélange CH₃COOH / EtOH (3/2 (v/v)), est ajoutée une solution aqueuse contenant le dérivé monoaldéhydique du POE. Le pH du mélange est alors ajusté à 5,1. Une solution aqueuse de cyanoborohydrure de sodium ou de picoline borane ou de borohydrure de sodium (agents réducteurs) peut être ajoutée. Le pH du milieu réactionnel est ajusté à 9 puis le dérivé CHI-POEPOP-POE est purifié et isolé.
Selon une autre variante de l'invention, le dérivé CHI-POEPOP-POE peut être obtenu par un procédé comprenant les étapes successives suivantes :
a'. Préparation d'un dérivé de chitosane, CHI-POE, à partir du chitosane natif par greffage de chaîne poly(oxyde d'éthylène), ladite chaîne ayant avantageusement une masse moléculaire comprise entre 1000 et 5000 g/mol; ledit dérivé CHI-POE devant avoir un degré de substitution supérieur à 0,1 ;
b'. Réaction du dérivé CHI-POE obtenu à l'étape a) avec le dérivé POEPOP-acétal selon l'invention préalablement hydrolysé pour conduire au dérivé recherché.

Le chitosane natif peut tout d'abord être modifié avec un dérivé aldéhydique du POE, obtenu par oxydation à l'aide du réactif de Dess-Martin (Dess-Martin periodinane (DMP)) de l'alcool correspondant commercial, conduisant à la formation d'une base de Schiff. Celle-ci peut être réduite en milieu aqueux homogène en présence d'un agent réducteur (picoline borane (PicBH₃) ou borocyanohydrure de sodium (NaCNBH₃) ou borohydrure de sodium (NaBH₄)) pour conduire à un dérivé CHI-POE, soluble dans l'eau à pH neutre. Celui-ci est purifié, par exemple par ultrafiltration. L'introduction de chaînes POEPOP sur le dérivé précédent, par le même procédé que celui décrit précédemment (greffage du POEPOP sur le chitosane) conduit au produit attendu. Outre via une oxydation du POE-OH par le réactif de Dess-Martin, les dérivés aldéhydiques du POE peuvent être préparés par d'autres méthodes bien connues de l'homme de l'art que, telles que par exemple une oxydation en présence d'acide acétique et de DMSO (N. Bhattarai, H. R. Ramay, J. Gunn, F. A. Matsen, M. Zhang, J. Controlled Release 103 (2005), 609-624) et J. M. Harris, E. C. Struck, M. G. Case, M. S. Paley, J. M. Vanalstine, D. E. Brooks, J. Polym. Part A. : Polym Chem. 22 (1984), 341-352).
La première variante consistant à greffer le POE directement sur le dérivé CHI-POEPOP présente l'avantage d'être plus simple à mettre en oeuvre pour la purification du produit final.

L'invention a pour quatrième objet un dérivé de chitosane CHI-POEPOP-POE susceptible d'être obtenu par le procédé selon l'invention.

L'invention concerne également une composition aqueuse comprenant au moins un dérivé de chitosane CHI-POEPOP-POE selon l'invention et une phase aqueuse. Le dérivé de chitosane CHI-POEPOP-POE est avantageusement présent en une quantité comprise entre 2 et 30 g/L. Le pH de ladite composition aqueuse varie avantageusement de 4 au pH physiologique, de préférence le pH est voisin du pH physiologique (pH physiologique ± 1), le dérivé chitosane CHI-POEPOP-POE présentant l'avantage d'être soluble dans l'eau au pH physiologique. La composition aqueuse selon l'invention peut par ailleurs comprendre un milieu cosmétiquement ou pharmaceutiquement acceptable.
L'invention a pour autre objet l'utilisation de la composition aqueuse selon l'invention pour la fabrication de gels par chauffage.
Les dérivés CHI-POEPOP-POE présentent, comme pour les dérivés CHI-POEPOP des propriétés thermogélifiantes intéressantes, à savoir lorsqu'ils sont mis en solution une transition sol-gel réversible et contrôlable. Les solutions aqueuses résultantes conduisent à des liquides hautement visqueux ou à des gels transparents par chauffage, en particulier à une température qui peut être proche de celle du corps humain. En outre, ces dérivés présentent l'avantage d'être solubles dans l'eau au pH physiologique facilitant leurs applications biomédicales. Ces systèmes, biorésorbables, peuvent ainsi être utilisés dans des formulations cosmétiques, pharmaceutiques ou biomédicales, qui peuvent être transparentes, pour en modifier les propriétés rhéologiques en fonction de la température. En particulier, ils peuvent être utilisés pour la fabrication d'hydrogels injectables.
Cette famille de dérivés thermogélifiants du chitosane solubles en milieu aqueux dont le pH varie de 4 à des valeurs de pH physiologique est susceptible de trouver des applications diverses dans les domaines cosmétique, pharmaceutique ou biomédical.

Les exemples qui suivent illustrent l'invention, mais ne sont pas limitatifs. Dans ces exemples, la concentration en polymère est exprimée en g/L ou en monomol/L, correspondant au nombre de moles d'unités de répétition dans 1 L de solution.

### Exemple 1 : Synthèse d'un dérivé POEPOP-acétal

A une solution de POEPOP (7,01g, 3,2 mmol), dans le DMF anhydre (300 mL) sous azote, l'anhydride succinique (0,336 g, 3,36 mmol) en solution dans le DMF anhydre (20 mL) est ajouté. Le milieu réactionnel est laissé sous agitation à température ambiante (et à pression atmosphérique) pendant 5 heures. Sont ajoutés ensuite successivement, le N-hydroxybenzotriazole (HOBt) (0,432 g, 3.2 mmol), solubilisé dans le DMF anhydre (10 mL), le 1-éthyl-3-[3-(diméthylamino)propyl]-carbodiimide (EDC) (0,675 g, 3,52 mmol) et la diisopropyléthylamine (DIEA) (0,600 mL, 3,52 mmol) solubilisés ensemble dans le DMF anhydre (10 mL) et enfin la 2,2-diméthoxyéthylamine (0,47g , 4,48 mmol). Le milieu réactionnel est laissé sous vive agitation pendant une nuit à température ambiante et à pression atmosphérique. Après évaporation du DMF, le produit est repris avec du dichlorométhane. La phase organique est lavée successivement avec de l'eau acidulée (pH 4), puis de l'eau. La phase organique est séchée sur sulfate de sodium, puis évaporée pour donner le dérivé acétal attendu avec un rendement de 86%.

### Exemple 2 : Synthèse d'un dérivé CHI-POEPOP avec un DS de 0,1

(ajout de 0,11 équivalent molaire de POEPOP par rapport au chitosane). La première étape consiste à déprotéger la fonction aldéhyde du POEPOP-acétal. Celui-ci (1,8 g, 0,66 mmol) est solubilisé dans un mélange TFA/H₂O (23 mL) dans les proportions (4/1) (v/v). Le milieu réactionnel est laissé sous vive agitation pendant une nuit à température ambiante et à pression atmosphérique. Le mélange TFA/H₂O est évaporé au rotavapor. L'aldéhyde ainsi obtenu (0,66 mmol) est solubilisé dans un mélange EtOH/ H₂O (1/1 (v/v), 15 mL) et le pH de la solution est ajusté à pH 5,1. Celle-ci est alors ajoutée au chitosane (1 g, 6 mmol), préalablement solubilisé dans un mélange CH₃COOH 0,2 M/ EtOH (3 :2 (v/v), 320 mL). Le pH du mélange est alors ajusté à 5,1 avec une solution d'hydroxyde de sodium (0,5 M). Une solution aqueuse (2 mL) de cyanoborohydrure de sodium (0,532 g, 7,2 mmol) est ajoutée. Après 24 h d'agitation à température ambiante et à pression atmosphérique, le pH du milieu réactionnel est ajusté à 9 conduisant à la précipitation du produit attendu. Ce dernier est alors lavé successivement avec des mélanges EtOH/ H₂O (3/2, 7/3, 4/1, 9/1 (v/v)) puis à l'éthanol. Le produit est filtré sur fritté 4 et séché à l'air libre. On obtient 1,6 g de dérivé CHI-POEPOP (Rdt 63 %). Le degré de substitution (déterminé par RMN ¹H à 25 °C) du dérivé est de 0,1.

### Exemple 3 : Synthèse d'un dérivé CHI-POEPOP-POE

Synthèse effectuée avec un POE de masse molaire 2000 g/mol, un CHI-POEPOP de DS 0,1 et on vise un DS en POE de ~ 0,25.
A une solution de monoéther méthylique de poly(oxyde d'éthylène) (2 g, 1 mmol) dans une solution de CH₂Cl₂/DMSO anhydre (97/3, (v/v), 100 mL), sous azote, est ajoutée lentement une solution de Dess-Martin Periodinane (DMP) (0,424 g, 1 mmol) dans le DMSO (4 mL). Le mélange réactionnel est laissé sous agitation pendant 2 h à température ambiante. Le milieu est concentré à l'évaporateur rotatif jusqu'à l'obtention d'une huile blanchâtre. L'huile résiduelle est précipitée dans l'éther éthylique (600 mL). Le produit brut est isolé par filtration sur fritté 4. Le dérivé monoaldéhydique du POE obtenu après filtration est séché sous pression réduite à 25°C pendant 2h.
A une solution de Chitosane-POEPOP de DS 0,1 (0,3 g ; 0,75 mmol) préalablement solubilisée dans un mélange CH₃COOH 0,2 M/ EtOH (3/2 (v/v), 80 mL), est ajoutée une solution aqueuse de POE oxydé (0,52 g, 0,26 mmol). Le pH du mélange est alors ajusté à 5,1 avec une solution d'hydroxyde de sodium (0,5 M). Une solution aqueuse (2 mL) de cyanoborohydrure de sodium (0,028 g, 0,375 mmol) est ajoutée. Après 24h d'agitation à température ambiante et à pression atmosphérique, le pH du milieu réactionnel est ajusté à 9. Le produit, qui ne précipite pas, est ensuite purifié par ultrafiltration en utilisant H₂O comment solvant de lavage (et une membrane Millipore YM 10 minimum préférentiellement 30 ou 100). Après lyophilisation, on obtient le dérivé CHI-POEPOP-POE (Rendement 66 %).

### Exemple 4 : comportement rhéologique des dérivés CHI-POEPOP en milieu aqueux

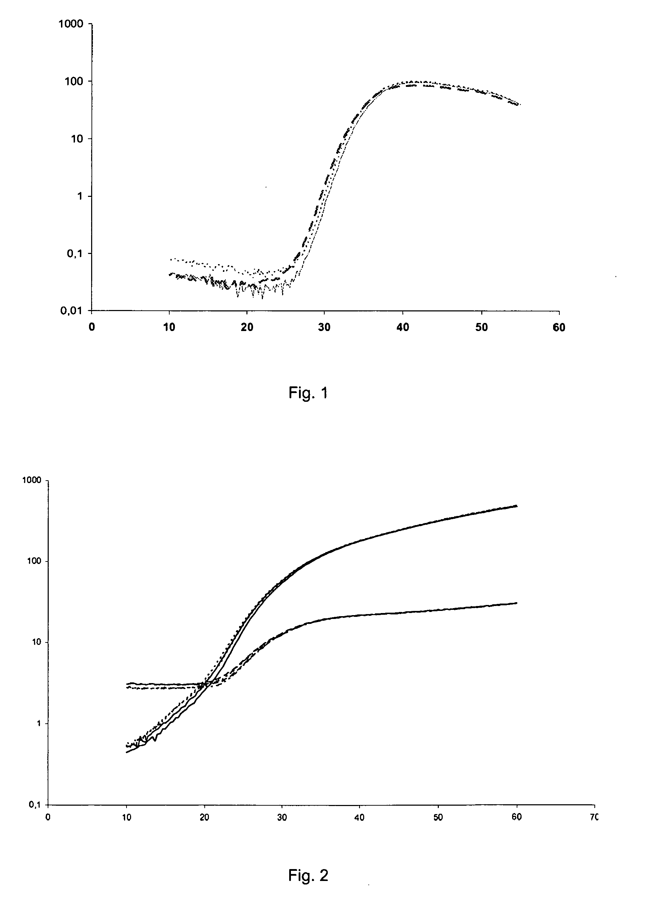

II a été vérifié que les solutions aqueuses comprenant les dérivés CHI-POEPOP, à une concentration de 10 g/L (0,024 monomol/L) dans AcOH 0,3M/AcONa 0,05 M, présentent une transition sol-gel qui est réversible.
Les résultats sont reportés sur la figure 1 qui représente l'évolution de la viscosité (Pa/s) de la solution aqueuse en fonction de la température (°C). Légende:
Aller 1: pointillés (·····)
Retour 1 : trait continu (------)
Aller 2: trait discontinu (- - -)
On constate donc que la transition sol-gel est réversible et reproductible.

### Exemple 5: comportement rhéologique des dérivés CHI-POEPOP-POE en milieu aqueux

La Figure 2 met en évidence la transition sol-gel réversible du dérivé CHI-POEPOP-POE (DS₂ = 0,26, DS₁ = 0,17) soluble en milieu aqueux à pH neutre. Les courbes reportées donnent la variation des modules de conservation (élastique) G' et de perte (visqueux) G" (Pa) en fonction de la température (°C) pour le copolymère thermoassociatif à base de chitosane CHI-POEPOP-POE, dans les conditions opératoires suivantes : fréquence fixée à 1 Hz ; vitesse de chauffage : 1°C/min; solvant : NaCl 0,1 M ; Cp = 40g/L (0,035 monomol/L) lors d'un premier chauffage et d'un deuxième chauffage.

### Légende de la figure 2 :

G', aller 1: pointillés (·····)
G", aller 1: un trait, un pointillé (-·-·-·-·)
G', aller 2 : trait continu (------)
G", aller 2: trait discontinu (- - -)
On constate donc que la transition sol-gel est reproductible.

### Exemple 6: comportement rhéologique des dérivés CHI-POEPOP et CHI-POEPOP-POE en milieu aqueux, en fonction de la température

### Mise en évidence du caractère thermogélifiants

Des mesures rhéologiques en écoulement et en régime dynamique ont permis de mettre clairement en évidence le comportement thermoassociatif des dérivés CHI-POEPOP et CHI-POEPOP-POE.

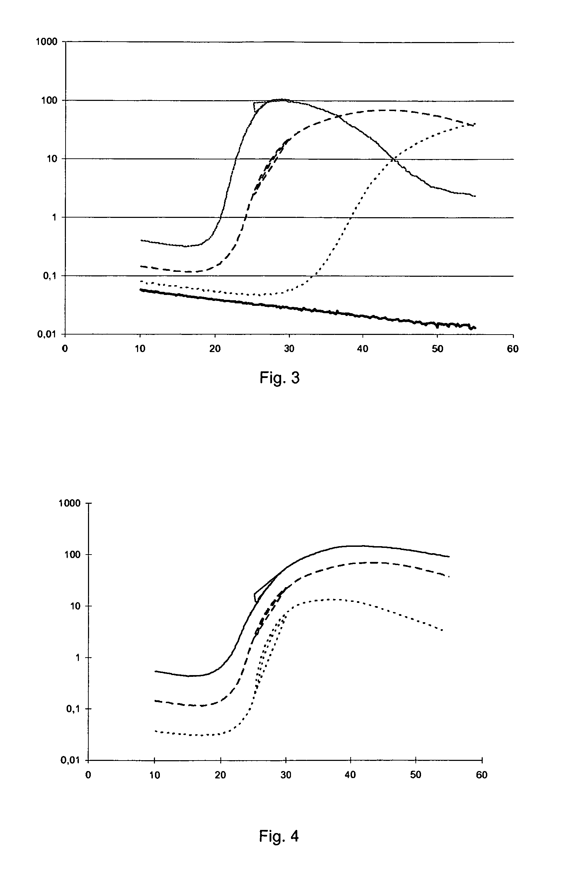

La figure 3 montre l'évolution de la viscosité en fonction de la température mesurée à partir de solution de dérivés CHI-POEPOP de DS₁ variable, dans les conditions opératoires suivantes : vitesse de cisaillement : 1s⁻¹; vitesse de chauffage : 1°C/min ; solvant : acide acétique (AcOH) 0,3 M / acétate de sodium (AcONa) 0,05 M; concentration en polymère = Cp = 0,024 monomol/L. Les courbes reportées donnent l'évolution de la viscosité (Pa.s) en fonction de la température (°C).

### Légende de la figure 3 :

CHI-POEPOP DS₁ = 0,17 : trait continu (-)
CHI-POEPOP DS₁ = 0,1 : trait discontinu (---)
CHI-POEPOP DS₁ = 0,04 : pointillés (·····)
CHI : trait continu gras (------)
On constate une augmentation remarquable de la viscosité d'un facteur compris entre 100 et 1000 en fonction du DS₁. La température de transition est étroitement liée au DS₁.
L'effet de la concentration en polymère sur la viscosification de la solution en fonction de la température est illustré dans la figure 4. La figure 4 représente la courbe de la viscosité (Pa.s) en fonction de la température (°C) pour l'échantillon CHI-POEPOP de DS₁ = 0,1, dans les conditions opératoires suivantes : vitesse de cisaillement : 1s⁻¹; vitesse de chauffage : 1°C/min ; solvant : AcOH 0,3 M/AcONa 0,05 M.

### Légende de la figure 4 :

CHI-POEPOP, DS₁ = 0,1, 15 g/L, 0,036 monomol/L: trait continu (------)
CHI-POEPOP, DS₁ = 0,1, 10 g/L, 0,024 monomol/L: trait discontinu (- - -)
CHI-POEPOP, DS₁ = 0,1, 5 g/L, 0,012 monomol/L: pointillés (·····)
Quelle que soit la concentration, le gain en viscosité atteint un facteur voisin de 1000. La température de transition reste sensiblement inchangée.

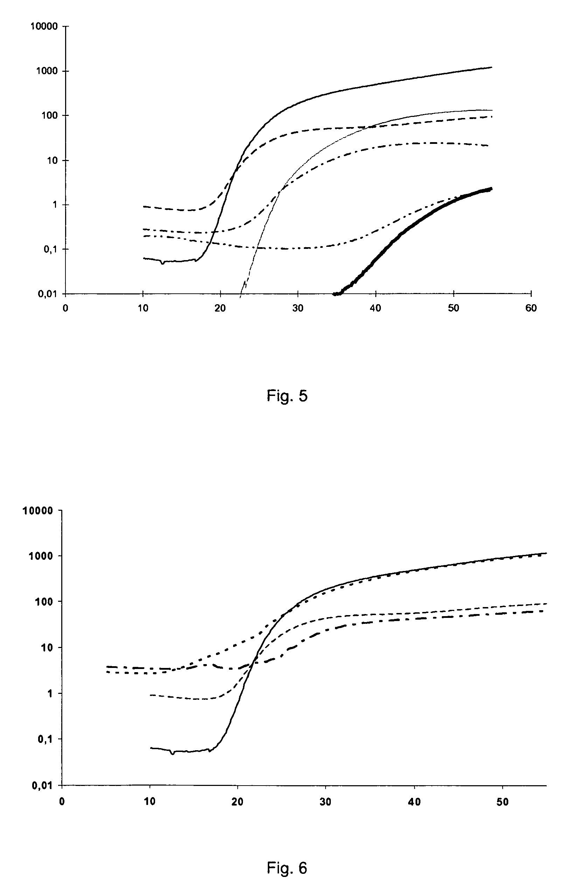

La figure 5 compare l'évolution des modules de conservation (élastique) G' et de perte (visqueux) G" en fonction de la température pour les différents CHI-POEPOP. L'intersection entre le module de conservation G' et le module de perte G" se situe à la température critique Tc. Cette température délimite deux domaines : dans la région à faible température (T<Tc) le caractère visqueux est prédominant, dans la région à haute température (T>Tc) le caractère élastique est prédominant. Les courbes reportées donnent la variation des modules G' et G" (Pa) en fonction de la température (°C) pour différents copolymères thermoassociatifs CHI-POEPOP dans les conditions opératoires suivantes : fréquence fixée à 1 Hz ; vitesse de chauffage : 1°C/min ; solvant: AcOH 0,3 M/AcONa 0,1 M ; Cp = 0,024 monomol/L.

### Légende de la figure 5 :

G', CHI-POEPOP DS₁ = 0,1: pointillés (·····)
G", CHI-POEPOP DS₁ = 0,1: un trait, un pointillé (-·-·-·-·)
G', CHI-POEPOP DS₁ = 0,17: trait continu (------)
G", CHI-POEPOP DS₁ = 0,17: trait discontinu (- - -)
G', CHI-POEPOP DS₁ = 0,04: trait continu gras (-------)
G", CHI-POEPOP DS₁ = 0,04: un trait, deux pointillés (-··-··)
Une augmentation importante des modules G' et G" en fonction de la température est observée pour les dérivés de DS₁ plus élevé (DS₁ = 0,17 et 0,1). A basse température, les composés en solution à pH 4 présentent un comportement de type solution visqueuse (G'<G") mais au-dessus de 25°C, ils adoptent un comportement de type gel (G'>G"). Le phénomène de thermo-association est donc bien mis en évidence pour ces dérivés.

### Exemple 7: Comparaison des comportements rhéologiques des dérivés CHI-POEPOP et de dérivés CHI- POEPOP-POE en milieu aqueux

Les comportements rhéologiques des dérivés CHIPOEPOP et CHI-POEPOP-POE en milieu aqueux ont été étudiés. Les conditions opératoires sont les suivantes :
- CHI-POEPOP de DS₁ 0,17 dans CH₃COOH 0,3M/CH₃COONa 0,1M (14,9g/L ou 0,024 monomol/L) et
- CHI-POEPOP-POE (DS_{POEPOP} (DS₁)= 0,18 et DS_{POE} (DS₂)= 0,28) dans un tampon phosphate (pH=7,4, [NaCl]=0,134M) (28,8g/L ou 0,025monomol/L);
- la fréquence est fixée à 1 Hz;
- la vitesse de chauffage est de 1 °C/min.
Les résultats sont reportés sur la figure 6 qui représente la variation des modules G' et G" (Pa) de la solution aqueuse en fonction de la température (°C). Légende:
G', CHI-POEPOP DSᵢ = 0,17: pointillés (·····)
G", CHI-POEPOP DS₁ = 0,17: un trait, un pointillé (-·-·-·-·)
G', CHI- POEPOP-POE DS₁ = 0,18: trait continu (-------)
G", CHI- POEPOP-POE DS₁ = 0,18: trait discontinu (- - -)
D'après la figure 6, on peut remarquer que les modules G' et G" sont plus élevés aux basses températures, dans le cas du dérivé CHI-POEPOP-POE. L'effet épaississant induit par la présence de groupements POE est également observé lorsqu'on compare la viscosité de solutions de CHI-POE et de chitosane natif dans les mêmes conditions de solvant. Cette différence est accentuée lorsque le dérivé CHI-POE est solubilisé, comme dans le cas présent pour le dérivé CHI-POEPOP-POE, dans le tampon phosphate à pH 7,4 (avec [NaCl]=0,134M), où les répulsions électrostatiques sont largement diminuées, favorisant ainsi les associations interchaînes. Par ailleurs, on peut noter que la présence de chaînes POE greffées entraîne l'apparition du point de croisement des courbes G' et G" à une température plus faible, mais n'a pas d'effet significatif sur la valeur des modules aux températures élevées. Ces résultats semblent donc indiquer que lors de l'augmentation de température, les greffons POEPOP s'agrègent entre eux et la présence de chaînes POE renforce le phénomène d'association.

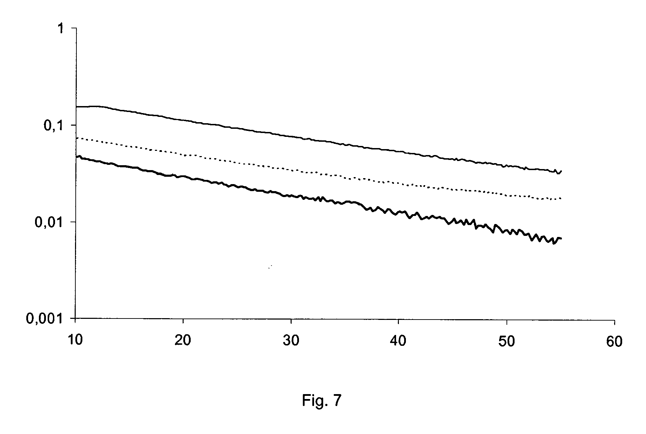

La figure 7 montre l'évolution de la viscosité en fonction du gradient de cisaillement pour une solution de CHI-POE à 17,7 g/L (0,025 monomol/L) dans différents solvants et le chitosane initial.

### Légende de la figure 7 : viscosité (Pa.s) en fonction de la température (°C)

CHI-POE PBS - tampon phosphate (pH=7,4, [NaCl]=0,134M): trait continu (-------)
CHI-POE acide - AcOH 0,3M/AcONa 0,05 M : pointillés (·····)
CHI natif acide - AcOH 0,3M/AcONa 0,05 M : trait continu gras (-**------**)
On constate des valeurs de viscosités plus élevées pour le dérivé CHI-POE. Pour ce dérivé, l'utilisation d'un tampon phosphate à pH 7,4 (avec [NaCl]=0,134M), où les répulsions électrostatiques sont largement diminuées, favorise les associations interchaînes.

## Revendications

1. Procédé de préparation de dérivé POEPOP-acétal thermosensible comprenant les étapes successives suivantes :
a. Réaction d'un ou plusieurs polyétheramines statistiques (POEPOP) de formule générique (I) 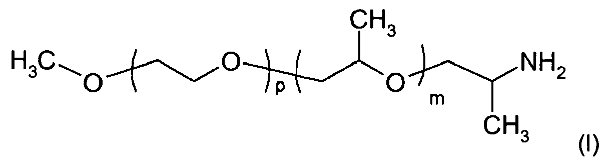 dans lequel p représente un nombre entier variant de 1 à 40, avantageusement de 3 à 29, et m représente un nombre entier variant de 1 à 40, avantageusement de 1 à 31,
avec l'anhydride succinique pour conduire à l'acide correspondant;
b. Couplage de l'acide obtenu suite à l'étape a) avec la 2,2-diméthoxyéthylamine pour conduire au dérivé POEPOP-acétal recherché.

2. Procédé de préparation selon la revendication 1, **caractérisé en ce que** le solvant de la réaction utilisé à l'étape a) est le DMF anhydre.

3. Procédé de préparation selon la revendication 1 ou 2, **caractérisé en ce que** suite à l'étape a) et préalablement à l'étape b), le milieu réactionnel est laissé sous agitation.

4. Procédé de préparation selon l'une quelconque des revendications précédentes, **caractérisé en ce que** le solvant de la réaction utilisé à l'étape b) est le DMF.

5. Procédé de préparation selon l'une quelconque des revendications précédentes, **caractérisé en ce que** la réaction de couplage, étape b), comprend également l'ajout de 1-éthyl-3-[3-(diméthylamino)propyl]-carbodiimide et de diisopropyléthylamine.

6. Procédé de préparation selon l'une quelconque des revendications précédentes, **caractérisé en ce que** les étapes a) et b) sont réalisées « en un seul pot ».

7. Dérivé POEPOP-acétal obtenu par le procédé selon l'une quelconque des revendications précédentes.

8. Procédé de synthèse de chitosane greffé comprenant (i) une étape d'hydrolyse du dérivé POEPOP-acétal selon la revendication 7 en dérivé POEPOP-CHO suivie (ii) d'une étape de greffage dudit dérivé POEPOP-CHO sur le chitosane ou un des ses dérivés par une réaction du type amination réductrice.

9. Procédé selon la revendication 8, **caractérisé en ce que** ledit chitosane ou un des ses dérivés répond à la formule (II) suivante : 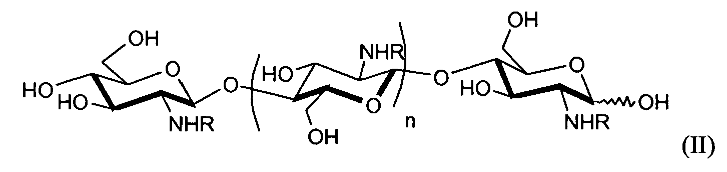 dans laquelle n varie de 60 à 6000 et R représente
- un atome d'hydrogène H, ou
- un radical acétyle COCH₃, le degré d'acétylation étant avantageusement compris entre 0 et 0, 5, plus avantageusement entre 0 et 0,2.

10. Procédé selon l'une quelconque des revendications 8 à 9, **caractérisée en ce qu'**il comprend une étape supplémentaire (iii) de fonctionnalisation du dérivé chitosane CHI-POEPOP obtenu suite à l'étape (ii) par un polymère.

11. Procédé selon la revendication 10, **caractérisé en ce que** le dérivé chitosane CHI-POEPOP est fonctionnalisé par un poly(oxyde d'éthylène) ayant avantageusement une masse moléculaire comprise entre 1000 et 5000 g/mol, le degré de substitution DS₂ étant supérieur à 0,1.

12. Dérivé de chitosane CHI-POEPOP-POE susceptible d'être obtenu par le procédé selon les revendications 10 ou 11.

13. Composition aqueuse comprenant au moins un dérivé de chitosane CHI-POEPOP-POE selon la revendication 12 et une phase aqueuse.

14. Composition aqueuse selon la revendication 13, **caractérisée en ce que** le dérivé de chitosane CHI-POEPOP-POE est présent en une quantité comprise entre 2 et 30 g/L.

15. Composition aqueuse selon l'une quelconque des revendications 13 et 14, **caractérisée en ce que** le pH de ladite composition aqueuse varie de 4 au pH physiologique.

16. Composition aqueuse selon l'une quelconque des revendications 13 à 15, comprenant par ailleurs un milieu cosmétiquement ou pharmaceutiquement acceptable.

17. Utilisation de la composition aqueuse selon l'une quelconque des revendications 13 à 16 pour la fabrication de gels par chauffage.

## Claims

1. A method for preparing a thermosensitive PEOPPO-acetal derivative comprising the following successive steps:
a. reaction of one or more random polyetheramines (PEOPPO) of generic formula (I) 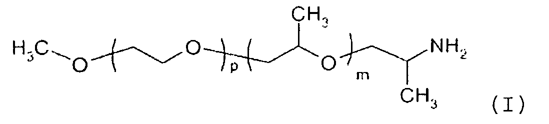 wherein p represents an integer varying from 1 to 40, advantageously from 3 to 29, and m represents an integer varying from 1 to 40, advantageously from 1 to 31,
with succinic anhydride in order to lead to the corresponding acid;
b. coupling of the acid obtained following step a) with 2,2-dimethoxyethylamine in order to lead to the sought PEOPPO-acetal derivative.

2. The preparation method according to claim 1, **characterized in that** the solvent of the reaction used in step a) is anhydrous DMF.

3. The preparation method according to claim 1 or 2, **characterized in that** following step a) and prior to step b), the reaction medium is left under stirring.

4. The preparation method according to any of the preceding claims, **characterized in that** the solvent of the reaction used in step b) is DMF.

5. The preparation method according to any of the preceding claims, **characterized in that** the coupling reaction, step b), also comprises the addition of 1-ethyl-3-[3-(dimethylamino)propyl]-carbodiimide and diisopropylethylamine.

6. The preparation method according to any of the preceding claims, **characterized in that** the steps a) and b) are carried out in a single pot.

7. A PEOPPO-acetal derivative obtained by the method according to any of the preceding claims.

8. A method for synthesis of grafted chitosan comprising (i) a step for hydrolyzing the PEOPPO-acetal derivative according to claim 7 into a PEOPPO-CHO derivative followed by (ii) a step for grafting said PEOPPO-CHO derivative on chitosan or one of its derivatives by a reaction of the reducing amination type.

9. The method according to claim 8, **characterized in that** said chitosan or one of its derivatives fits the following formula (II): 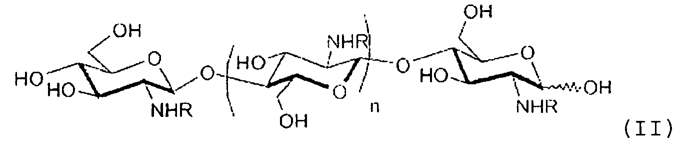 wherein n varies from 60 to 6,000 and R represents
- a hydrogen atom H, or
- an acetyl radical COCH₃, the acetylation degree being advantageously comprised between 0 and 0.5, more advantageously between 0 and 0.2.

10. The method according to either of claims 8 and 9, **characterized in that** it comprises an additional step (iii) for functionalizing the CHI-PEOPPO chitosan derivative obtained subsequently to step (ii) by a polymer.

11. The method according to claim 10, **characterized in that** the CHI-PEOPPO chitosan derivative is functionalized by a poly(ethylene oxide) advantageously having a molecular mass comprised between 1,000 and 5,000 g/mol, the degree of substitution DS₂ being greater than 0.1.

12. A CHI-PEOPPO-PEO chitosan derivative which may be obtained by a method according to claims 10 or 11.

13. An aqueous composition comprising at least one CHI-PEOPPO-PEO chitosan derivative according to claim 12 and an aqueous phase.

14. The aqueous composition according to claim 13, **characterized in that** the CHI-PEOPPO-PEO chitosan derivative is present in an amount comprised between 2 and 30 g/L.

15. The aqueous composition according to either of claims 13 and 14, **characterized in that** the pH of said aqueous composition varies from 4 to the physiological pH.

16. The aqueous composition according to any of claims 13 to 15, moreover comprising a cosmetically or pharmaceutically acceptable medium.

17. The use of the aqueous composition according to any of claims 13 to 16, for making gels by heating.

## Patentansprüche

1. Verfahren zur Herstellung eines wärmeempfindlichen PEO-PPO-Acetal-Derivats, umfassend die folgenden aufeinanderfolgenden Schritte:
a) Umsetzen eines oder mehrerer statistischer Polyetheramine (PEO-PPO) der allgemeinen Formel (I) 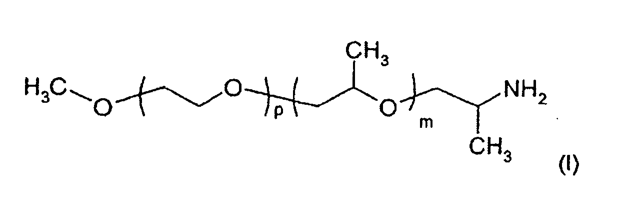 wobei p eine ganze Zahl von 1 bis 40, vorzugsweise 3 bis 29, darstellt und m eine ganze Zahl von 1 bis 40, vorzugsweise 1 bis 31, darstellt,
mit Bernsteinsäureanhydrid, um die entsprechende Säure zu ergeben;
b) Kuppeln der im Anschluss an Schritt a) erhaltenen Säure mit 2,2-Dimethoxyethylamin, um das gewünschte PEO-PPO-Acetal-Derivat zu ergeben.

2. Herstellungsverfahren nach Anspruch 1, **dadurch gekennzeichnet, dass** das in Schritt a) verwendete Reaktionslösungsmittel wasserfreies DMF ist.

3. Herstellungsverfahren nach Anspruch 1 oder 2, **dadurch gekennzeichnet, dass** das Reaktionsmedium im Anschluss an Schritt a) und vor Schritt b) rühren gelassen wird.

4. Herstellungsverfahren nach einem der vorhergehenden Ansprüche, **dadurch gekennzeichnet, dass** das in Schritt b) verwendete Reaktionslösungsmittel DMF ist.

5. Herstellungsverfahren nach einem der vorhergehenden Ansprüche, **dadurch gekennzeichnet, dass** die Kupplungsreaktion, Schritt b), außerdem die Zugabe von 1-Ethyl-3-[3-(Dimethylamino)propyl]carbodiimid und Diisopropylethylamin umfasst.

6. Herstellungsverfahren nach einem der vorhergehenden Ansprüche, **dadurch gekennzeichnet, dass** die Schritte a) und b) "in einem einzigen Topf" durchgeführt werden.

7. PEO-PPO-Acetal-Derivat, erhalten durch das Verfahren nach einem der vorhergehenden Ansprüche.

8. Verfahren zur Synthese von gepfropftem Chitosan, umfassend (i) einen Schritt der Hydrolyse des PEO-PPO-Acetal-Derivats nach Anspruch 7 zu einem PEO-PPO-CHO-Derivat, gefolgt von (ii) einem Schritt des Pfropfens des PEO-PPO-CHO-Derivats auf das Chitosan oder eines seiner Derivate durch eine reduktive Aminierungsreaktion.

9. Verfahren nach Anspruch 8, **dadurch gekennzeichnet, dass** das Chitosan oder eines seiner Derivate die folgende Formel (II) aufweist: 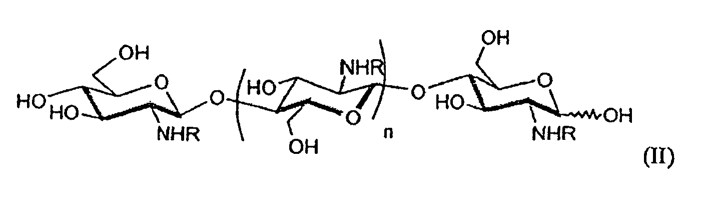 wobei n im Bereich von 60 bis 6000 liegt und R
- ein Wasserstoffatom H oder
- einen Acetylrest COCH₃, wobei der Acetylierungsgrad vorzugsweise zwischen 0 und 0,5, stärker bevorzugt zwischen 0 und 0,2 liegt,
bedeutet.

10. Verfahren nach einem der Ansprüche 8 bis 9, **dadurch gekennzeichnet, dass** es einen zusätzlichen Schritt (iii) der Funktionalisierung des im Anschluss an Schritt (ii) erhaltenen Chitosan-Derivats CHI-PEO-PPO mit einem Polymer umfasst.

11. Verfahren nach Anspruch 10, **dadurch gekennzeichnet, dass** das Chitosan-Derivat CHI-PEO-PPO mit einem Polyethylenoxid funktionalisiert wird, das vorzugsweise ein Molekulargewicht zwischen 1000 und 5000 g/mol aufweist, wobei der DS₂-Substitutionsgrad größer als 0,1 ist.

12. Chitosan-Derivat CHI-PEO-PPO-PEO, erhältlich durch das Verfahren nach den Ansprüchen 10 oder 11.

13. Wässrige Zusammensetzung, umfassend mindestens ein Chitosan-Derivat CHI-PEO-PPO-PEO nach Anspruch 12 und eine wässrige Phase.

14. Wässrige Zusammensetzung nach Anspruch 13, **dadurch gekennzeichnet, dass** das Chitosan-Derivat CHI-PEO-PPO-PEO in einer Menge zwischen 2 und 30 g/l vorliegt.

15. Wässrige Zusammensetzung nach einem der Ansprüche 13 und 14, **dadurch gekennzeichnet, dass** der pH der wässrigen Zusammensetzung im Bereich von 4 bis physiologischem pH liegt.

16. Wässrige Zusammensetzung nach einem der Ansprüche 13 bis 15, ferner umfassend ein kosmetisch oder pharmazeutisch verträgliches Medium.

17. Verwendung der wässrigen Zusammensetzung nach einem der Ansprüche 13 bis 16 zur Herstellung von Gelen durch Erwärmung.
